Europäisches Patentamt
European Patent Office
Office européen des brevets

(11) Veröffentlichungsnummer: 0 158 248
A2

# (12) EUROPÄISCHE PATENTANMELDUNG

(21) Anmeldenummer: 85103841.4

(22) Anmeldetag: 29.03.85

(51) Int. Cl.⁴: C 07 D 251/46
A 01 N 47/36
//C07C143/83

(30) Priorität: 11.04.84 JP 70909/84

(43) Veröffentlichungstag der Anmeldung:
16.10.85 Patentblatt 85/42

(84) Benannte Vertragsstaaten:
BE CH DE FR GB IT LI NL

(71) Anmelder: NIHON TOKUSHU NOYAKU SEIZO K.K.
No.4, 2-chome, Nihonbashi Honcho Chuo-ku
Tokyo 103(JP)

(72) Erfinder: Shiokawa, Kozo
210-6, Shukugawara Tama-ku
Kawasaki-shi Kanagawa-ken(JP)

(72) Erfinder: Kagabu, Shinzo
432-131-105, Terada-machi
Hachioji-shi Tokyo(JP)

(72) Erfinder: Moriya, Koichi
39-15, Namiki-cho
Hachioji-shi Tokyo(JP)

(74) Vertreter: Schumacher, Günter, Dr. et al,
c/o Bayer AG Konzernverwaltung RP Patentabteilung
D-5090 Leverkusen 1 Bayerwerk(DE)

## (54) Verfahren zur Herstellung von Biphenylylsulfonylharnstoff- Derivaten, Zwischenprodukte für diese sowie Verfahren für die Herstellung der Zwischenprodukte.

(57) Man erhält die herbizid wirksamen Biphenylylsulfonylharnstoff-Derivate der allgemeinen Formel I

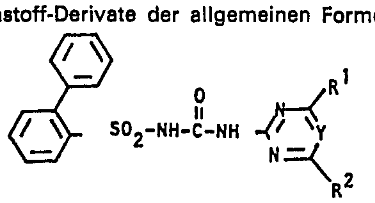

..... (I)

in der

R¹ und R² jeweils eine niedere Alkyl-Gruppe oder eine niedere Alkoxy-Gruppe bezeichnen und

Y CH oder N bezeichnet,

in guten Ausbeuten und hoher Reinheit, indem man Biphenylylsulfonylcarbamate der allgemeinen Formel II

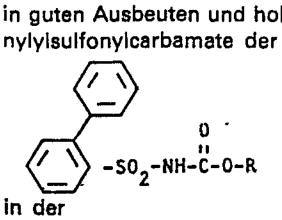

..... (II)

in der

R eine niedere Alkyl-Gruppe oder eine Aryl-Gruppe bezeichnet, mit einem heterocyclischen Amin der allgemeinen Formel (III)

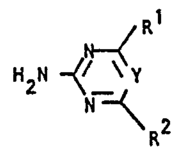

umsetzt. Die Zwischenprodukte der Formel (II) sind wertvolle neue Verbindungen; ein Verfahren zu ihrer Herstellung, gekennzeichnet durch die Umsetzung von 2-Phenylbenzolsulfonamid mit entsprechenden Kohlensäure-Derivaten der Formel R' −CO−OR (worin R' für Cl oder −OR steht), wird ebenfalls angegeben.

EP 0 158 248 A2

0158248

NIHON TOKUSHU NOYAKU SEIZO K.K., Tokyo/Japan

Bi-Ma
IVa(ZP)

# Verfahren zur Herstellung von Biphenylylsulfonylharnstoff-Derivaten, Zwischenprodukte für diese sowie Verfahren für die Herstellung der Zwischenprodukte

Die vorliegende Erfindung betrifft ein neues Verfahren zur Herstellung von Biphenylylsulfonylharnstoff-Derivaten, neue Zwischenprodukte für diese und ein Verfahren zur Herstellung dieser Zwischenprodukte.

Insbesondere betrifft die vorliegende Erfindung ein Verfahren zur Herstellung von Biphenylylsulfonylharnstoff-Derivaten der allgemeinen Formel (I)

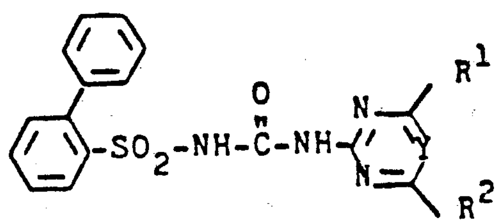

..... (I)

in der

$R^1$ und $R^2$ jeweils eine niedere Alkyl-Gruppe oder eine niedere Alkoxy-Gruppe bezeichnen und

Y CH oder N bezeichnet,

welches dadurch gekennzeichnet ist, daß man eine Verbindung der allgemeinen Formel (II)

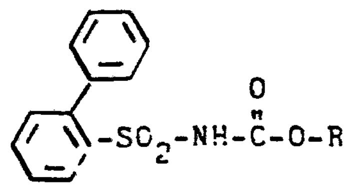

..... (II)

,

in der
R eine niedere Alkyl-Gruppe oder eine Aryl-Gruppe bezeichnet, mit einer Verbindung der allgemeinen Formel (III)

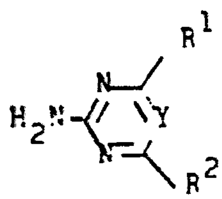

..... (III)

,

in der
$R^1$, $R^2$ und Y die im Vorstehenden angegebenen Bedeutungen haben, umsetzt.

Die Biphenylylsulfonylcarbamat-Derivate der oben angegebenen allgemeinen Formel (II) sind neue Verbindungen, die in der bekannten, vor der Einreichung der vorliegenden Anmeldung veröffentlichten Literatur nicht beschrieben sind.

Die Verbindungen der allgemeinen Formel (II) können mittels des nachstehenden Verfahrens synthetisiert werden, auf das sich die vorliegende Erfindung ebenfalls erstreckt.

Das Verfahren zur Herstellung der Biphenylylsulfonylcarbamat-Derivate der nachstehenden Formel (II)

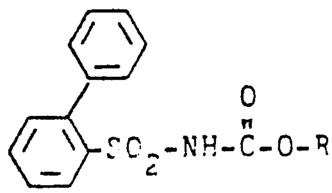 ..... (II) ,

in der
R eine niedere Alkyl-Gruppe oder eine Aryl-Gruppe bezeichnet, ist dadurch gekennzeichnet, daß man 2-Phenylbenzolsulfonamid der folgenden Formel (V)

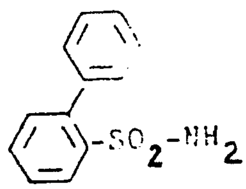 ..... (V)

mit einer Verbindung der folgenden Formel (IV)

$$R'-\overset{O}{\overset{\parallel}{C}}-O-R \quad ..... \text{(IV)}$$ ,

in der R die im Vorstehenden angegebene Bedeutung hat und R' ein Chlor-Atom oder die Gruppe -O-R bezeichnet, in der R die im Vorstehenden angegebene Bedeutung hat, umsetzt.

Seitens der Anmelderin wurden Untersuchungen mit dem Ziel eines neuen Verfahrens zur Herstellung von Biphenylylsulfonylharnstoff-Derivaten (I) durchgeführt, die ausgezeichnete selektive herbizide Wirkung besitzen und in der JP-OS 123 168/1982 der Anmelderin offenbart sind. Nunmehr wurde gefunden, daß die Verbindungen der Formel (I) in einfacher Weise in hoher Reinheit und hohen Ausbeuten mit Hilfe eines anderen, als des in der oben

zitierten JP-OS beschriebenen, Verfahrens hergestellt werden können, nämlich durch Reaktion der Biphenylylsulfonylcarbamat-Derivate der Formel (II) mit den Verbindungen der oben bezeichneten Formel (III).

In diesem neuen Verfahren sind die als Zwischenprodukte verwendeten Biphenylylsulfonylcarbamat-Derivate der Formel (II) neue Verbindungen, die in der bekannten, vor der Einreichung der vorliegenden Anmeldung veröffentlichten Literatur nicht beschrieben sind, und industriell wertvolle Zwischenprodukte bei der Synthese der wertvollen Verbindungen der Formel (I) sowie auch bei anderen Synthesen. Es wurde gefunden, daß diese Zwischenprodukte in einfacher Weise durch Reaktion von 2-Phenylbenzolsulfonamid der Formel (V) mit den Verbindungen der oben bezeichneten Formel (IV) hergestellt werden können.

In dem in der Beschreibung der oben genannten JP-OS 123 168/1982 erläuterten Verfahren zu Herstellung der Biphenylylsulfonylharnstoff-Derivate wird Sulfonylisocyanat als Zwischenprodukt benutzt. Die Herstellung des Sulfonylisocyanats ist jedoch industriell mit dem Nachteil behaftet, daß Phosgen oder ein Derivat desselben, Chlorameisensäure-trichloromethylester, verwendet werden müssen, die Probleme bezüglich ihrer Toxizität aufwerfen und ungünstig zu handhaben sind. Weiterhin ist zur Erzielung günstiger Ergebnisse eine Reaktionstemperatur nicht unbeträchtlicher Höhe zweckmäßig, zum Beispiel 120°C bis 130°C. Ein weiterer Nachteil liegt darin, daß zur Gewinnung des gewünschten Sulfonlyisocyanats in effizienter Reaktionsführung die Verwendung eines Katalysators wie 1,4-Diazabicyclo/¯2,2,2_7octan notwendig ist.

Die Untersuchungen seitens der Anmelderin haben nunmehr ergeben, daß ausgezeichnete, für die praktische Durchführung im industriellen Rahmen geeignete Verfahren verfügbar gemacht werden können, mit deren Hilfe die angeführten Nachteile des Standes der Technik überwunden werden können und durch die die neuen Verbindungen der Formel (II) leicht und quantitativ in einstufiger Reaktion bei Raumtemperatur aus der Verbindung der Formel (V) und den Verbindungen der Formel (IV) hergestellt werden können und dann durch Reaktion der Verbindungen der Formel (II) mit Verbindungen der Formel (III) die Biphenylylsulfonylharnstoff-Derivate der Formel (I) einfach und sicher in hoher Reinheit und hohen Ausbeuten hergestellt werden können.

Weiterhin wurde gefunden, daß die Biphenylylsulfonylcarbamat-Derivate der Formel (II) gemäß der vorliegenden Erfindung neue Verbindungen sind, die nicht nur industriell als Zwischenprodukte für die Herstellung der Verbindungen der Formel (I) und als Zwischenprodukte für andere Synthesen brauchbar sind, sondern auch selbst eine gewisse physiologische Aktivität besitzen (z.B. als Agrochemikalien).

Ziel der vorliegenden Erfindung ist dementsprechend, ein neues Verfahren zur Herstellung der Biphenylylsulfonylharnstoff-Derivate der Formel (I), die Biphenylylsulfonylcarbamat-Derivate der Formel (II) sowie ein Verfahren zur Herstellung der Carbamat-Derivate verfügbar zu machen.

Die vorbezeichneten und andere Ziele und Vorteile werden in der folgenden Beschreibung näher erläutert.

Das Verfahren zur Herstellung der Biphenylylsulfonylharnstoff-Derivate (I) läßt sich durch das nachstehende Reaktionsschema darstellen:

Verfahren i)

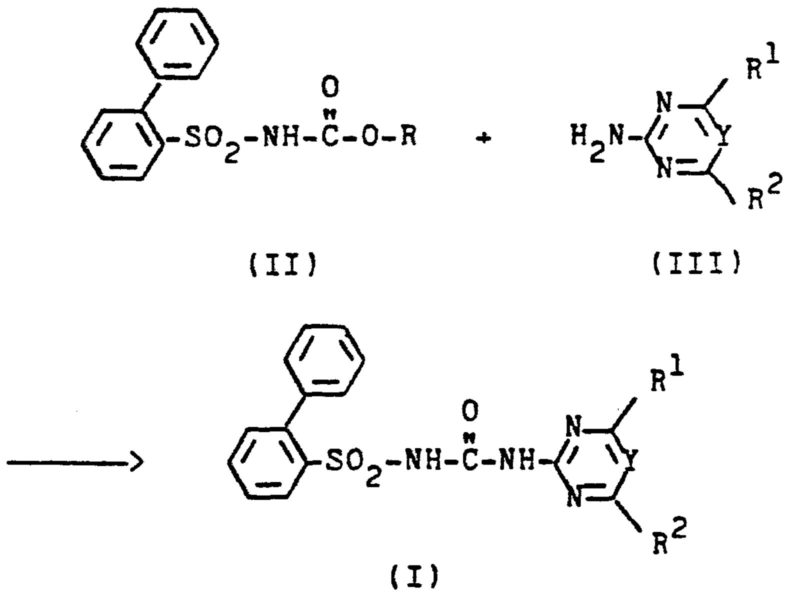

(II) (III)

(I)

In den Formeln haben R, $R^1$, $R^2$ und Y die im Vorstehenden angegebenen Bedeutungen.

Im vorstehenden Reaktionsschema bezeichnet R in der Verbindung der Formel (II) eine niedere Alkyl-Gruppe oder eine Aryl-Gruppe. Spezielle Beispiele sind niedere Alkyl-Gruppen wie Methyl, Ethyl, Propyl, Isopropyl und n-(iso-, sec- oder tert-)-Butyl und Aryl-Gruppen wie Phenyl, Naphthyl, Methylphenyl und Chlorophenyl.

Im vorstehenden Reaktionsschema bezeichnen $R^1$ und $R^2$ in den Verbindungen der Formeln (III) und (I) jeweils eine niedere Alkyl- oder Alkoxy-Gruppe. Speziell zu nennen sind die gleichen niederen Alkyl-Gruppen, wie

sie oben beispielhaft genannt sind, und niedere Alkoxy-Gruppen mit den gleichen niederen Alkyl-Gruppen, wie sie oben beispielhaft genannt sind.

Spezielle Beispiele für die Biphenylylsulfonylcarbamat-Derivate der allgemeinen Formel (II) als Ausgangsmaterial umfassen Phenyl-2-biphenylylsulfonylcarbamat, Methyl-2-biphenylylsulfonylcarbamat, Ethyl-2-biphenylylsulfonylcarbamat, 2-Tolyl-2-biphenylylsulfonylcarbamat, 4-Tolyl-2-biphenylylsulfonylcarbamat, α-Naphthyl-2-biphenylylsulfonylcarbamat und 4-Chlorophenyl-2-biphenylylsulfonylcarbamat.

Spezielle Beispiele für die Verbindung der allgemeinen Formel (III), die in gleicher Weise ein Ausgangsstoff ist, umfassen 2-Amino-4,6-dimethoxy-1,3,5-triazin, 2-Amino-4,6-dimethyl-1,3,5-triazin, 2-Amino-4-methoxy-6-methyl-1,3,5-triazin, 2-Amino-4,6-dimethylpyrimidin, 2-Amino-4-methoxy-6-methylpyrimidin und 2-Amino-4,6-dimethoxypyrimidin.

Das vorgenannte Verfahren wird durch das folgende typische Beispiel im einzelnen beschrieben.

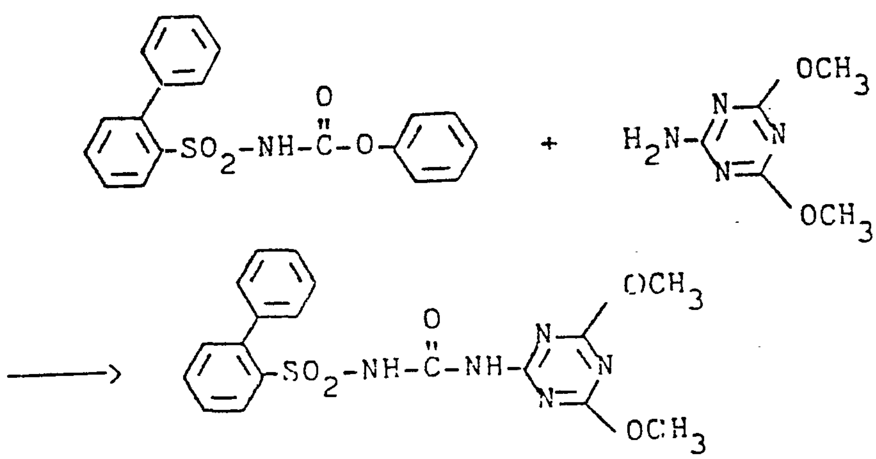

Zweckmäßigerweise kann das vorstehende Verfahren gemäß der vorliegenden Erfindung unter Verwendung eines Lösungsmittels oder Verdünnungsmittels durchgeführt werden. Zu diesem Zweck können sämtliche inerten Lösungsmittel oder Verdünnungsmittel verwendet werden.

Beispiele für solche Lösungsmittel oder Verdünnungsmittel umfassen aliphatische, alicyclische und aromatische Kohlenwasserstoffe (die gegebenenfalls chloriert sein können), wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Die vorstehende Reaktion kann in Gegenwart eines säurebindenden Mittels durchgeführt werden. Beispiele für solche säurebindenden Mittel sind die Hydroxide, Carbonate, Hydrogencarbonate und Alkoholate von Alkalimetallen sowie tertiäre Amine wie Triethylamin, Diethylanilin und Pyridin, die im allgemeinen verwendet werden.

Das obige Verfahren kann innerhalb eines breiten Temperaturbereichs durchgeführt werden. Beispielsweise kann es bei einer Temperatur zwischen etwa -20°C und

dem Siedepunkt der Mischung, vorzugsweise bei einer Temperatur zwischen 0°C und etwa 100°C, durchgeführt werden. Zweckmäßigerweise wird die Reaktion unter Atmosphärendruck durchgeführt, jedoch ist es ebenfalls möglich, bei erhöhtem oder vermindertem Druck zu arbeiten.

Die Biphenylylsulfonylcarbamat-Derivate der allgemeinen Formel (II) gemäß der vorliegenden Erfindung können auch mittels des folgenden Verfahrens hergestellt werden.

<u>Verfahren ii)</u>

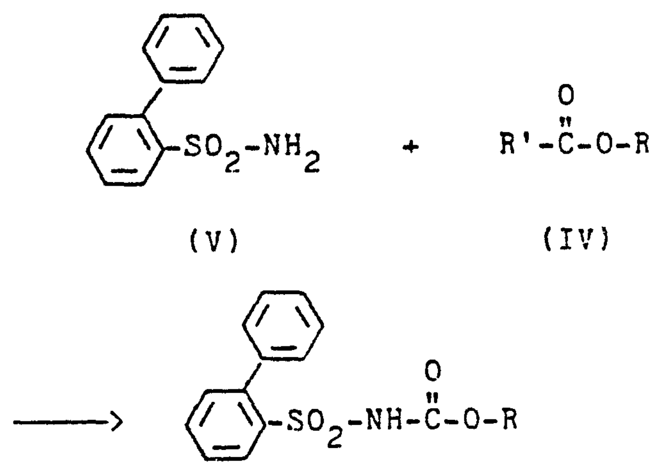

(V) (IV)

(II)

In den vorstehenden Formeln haben R und R' die im Vorstehenden angegebenen Bedeutungen.

Im vorstehenden Reaktionsschema können Beispiele für R die gleichen Substituenten sein, die oben für das Verfahren i) genannt wurden. R' bezeichnet ein Chlor-Atom oder die Gruppe -O-R, in der R die oben angegebene Bedeutung hat.

In dem Verfahren zur Herstellung der Biphenylylsulfonylcarbamat-Derivate der allgemeinen Formel (II) gemäß der vorliegenden Erfindung nach dem obigen Reaktionsschema zählen zu speziellen Beispielen für die Verbindung der allgemeinen Formel (IV) als Ausgangsmaterial Diphenylcarbonat, Chlorameisensäure-methylester, Chlorameisensäure-ethylester, Chlorameisensäure-phenylester, Dimethylcarbonat, Diethylcarbonat, Di(α-naphthyl)carbonat, Di-2-tolylcarbonat, Di-4-tolylcarbonat und Bis(4-chlorophenyl)carbonat.

Durch das folgende typische Beispiel wird das Verfahren zur Herstellung der Verbindungen der Formel (II) im einzelnen beschrieben.

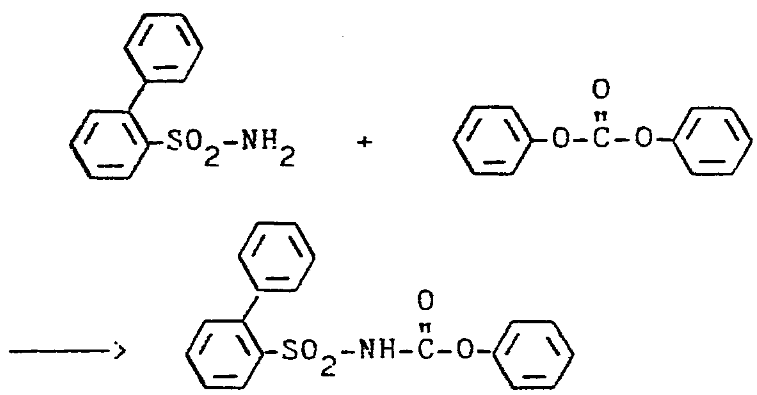

Zur Durchführung des vorstehenden Verfahrens können zweckmäßigerweise die gleichen inerten Lösungsmittel oder Verdünnungsmittel, wie sie oben beispielhaft angegeben sind, verwendet werden, und die gewünschte Verbindung kann in hoher Reinheit und in hoher Ausbeute erhalten werden.

Das obige Verfahren kann innerhalb eines breiten Temperaturbereichs durchgeführt werden, im allgemeinen bei

einer Temperatur zwischen etwa -20°C und dem Siedepunkt der Mischung, vorzugsweise zwischen 0°C und etwa 100°C. Zweckmäßigerweise wird die Reaktion unter Atmosphärendruck durchgeführt, jedoch ist es ebenfalls möglich, bei erhöhtem oder vermindertem Druck zu arbeiten.

Die vorliegende Erfindung wird durch die folgenden Beispiele im einzelnen erläutert, ist jedoch nicht auf diese speziellen Beispiele allein beschränkt.

## Beispiel 1

Synthese der Verbindungen der allgemeinen Formel (II) der vorliegenden Erfindung:

Natriumhydrid (2,6 g) wurde zu trockenem Dimethylformamid (200 ml) hinzugefügt, und unterhalb von 10°C wurde eine Lösung von 2-Phenylbenzolsulfonamid (23,3 g) in Dimethylformamid (50 ml) dazugegeben. Die Mischung wurde dann 1 h bei Raumtemperatur gerührt, und Diphenylcarbonat (21,4 g) wurde bei Raumtemperatur zugesetzt. Die Mischung wurde nochmals 1 h bei Raumtemperatur gerührt. Danach wurde die gesamte Mischung in Eiswasser gegeben, mit Salzsäure angesäuert und mit Ethylacetat extrahiert. Die organische Schicht wurde entwässert, und Ethylacetat wurde unter vermindertem Druck abdestilliert, wonach Phenyl-N-(2-biphenylylsulfonyl)carbamat (Feststoff, 35 g) als gewünschte Verbindung der nachstehenden Formel erhalten wurde; Schmp. 114-117°C.

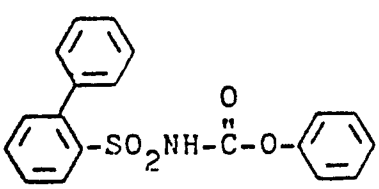

(Verbindung Nr. 1)

Nach dem gleichen Verfahren wie in Beispiel 1 wurden die in der folgenden Tabelle aufgeführten Verbindungen umgesetzt, wodurch die in der folgenden Tabelle aufgeführten Verbindungen der allgemeinen Formel (II) gemäß der vorliegenden Erfindung gebildet wurden.

Tabelle 1

| Ausgangsstoff | Ausgangsstoff | Produkt | (Verbindung Nr.) |
|---|---|---|---|
| 2-Phenylbenzol-sulfonamid | Chlorameisensäuremethylester | Methyl-N-(2-biphenylylsulfonyl)-carbamat (Schmp. 156-158°C) | Nr. 2 |
| 2-Phenylbenzol-sulfonamid | Di-α-naphthyl-carbonat | α-Naphthyl-N-(2-biphenylylsulfonyl)-carbamat | Nr. 3 |
| 2-Phenylbenzol-sulfonamid | Di-2-tolyl-carbonat | 2-Tolyl-N-(2-biphenylylsulfonyl)-carbamat | Nr. 4 |
| 2-Phenylbenzol-sulfonamid | Bis(4-chloro-phenyl)carbonat | 4-Chloro-N-(2-biphenylylsulfonyl)-carbamat | Nr. 5 |
| 2-Phenylbenzol-sulfonamid | Diethyl-carbonat | Ethyl-N-(2-biphenylylsulfonyl)-carbamat (Schmp. 147-150°C) | Nr. 6 |

Im Folgenden wird ein Beispiel für die Synthese der Verbindung der allgemeinen Formel (I) unter Verwendung von Phenyl-N-(2-biphenylylsulfonyl)carbamat, das nach dem in Beispiel 1 erläuterten Verfahren hergestellt wurde, angegeben.

Beispiel 2

Phenyl-N-(2-biphenylylsulfonyl)carbamat (35,3 g) und 2-Amino-4,6-dimethoxy-1,3,5-triazin (15,6 g) wurden zu Dioxan (200 ml) hinzugegeben, und die Mischung wurde 2 h unter Rühren zum Rückfluß erhitzt. Die Reaktionsmischung wurde auf Raumtemperatur abgekühlt, und die ausgeschiedenen Kristalle wurden durch Filtration gesammelt. Die Kristalle wurden mit Diethylether gewaschen und getrocknet, wonach der gewünschte 1-(2-Biphenylylsulfonyl)-3-(4,6-dimethoxytriazin-2-yl)harnstoff (36 g) der nachstehenden Formel erhalten wurde; Schmp. 175-180°C.

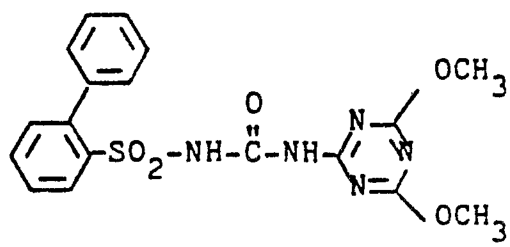

(Verbindung A)

Die biologische Wirkung der in Beispiel 2 synthetisierten Verbindung A wird in dem folgenden Beispiel 3 aufgezeigt.

Beispiel 3

Wagner-Töpfe (2 dm²) wurden mit Reis-Kulturboden gefüllt, und zwei Reis-Setzlinge im 2- bis 3-Blattstadium (Höhe etwa 10 cm) wurden in jeden Topf umgepflanzt. Samen von Hühnerhirse (Panicum crus-galli), Riedgras (Cyperus microiria), Monochoria (Monochoria vaginalis),

Simse (Scirpus juncoides) und breitblättrigen Unkräutern, kleine Stücke von Nadel-Sumpfbinse (Eleocharis acicularis) und Knollen von Cypergras (Cyperus serotinus) Urikawa (Pfeilkraut; Sagittaria pygmaea) wurden eingesetzt, und die Töpfe wurden in feuchtem Zustand gehalten. Als die Hühnerhirse etwa bis zum 2-Blatt-Stadium gewachsen war (etwa 7 bis 9 Tage nach der Einsaat), wurden die Töpfe mit Wasser bis zu einer Tiefe von etwa 6 cm gefüllt, und eine vorher festgelegte Menge der Verbindung A wurde in Form einer Emulsion mittels einer Pipette jedem der Töpfe zugegeben. Nach der Behandlung wurde das Wasser mit einer Geschwindigkeit von 2 bis 3 cm pro Tag aus den Töpfen heraussickern gelassen, und danach wurde die Wassertiefe in den Töpfen bei etwa 3 cm gehalten. In der vierten Woche nach der chemischen Behandlung wurden die herbizide Wirkung und der Grad der Phytotoxizität bewertet. Dabei wurde gefunden, daß die Verbindung A eine herbizide Wirkung von 100 % bei jedem der Unkräuter ausübte, wenn sie in einer Menge von 0,2 kg/ha (Menge der aktiven Verbindung) aufgebracht wurde; andererseits wurde keinerlei Phytotoxizität gegenüber Reis beobachtet.

## Patentansprüche

1. Verfahren zur Herstellung eines Biphenylylsulfonylharnstoff-Derivats der nachstehenden Formel (I)

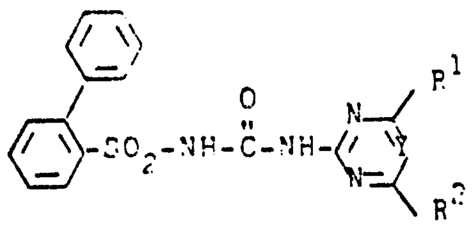

..... (I) ,

in der

$R^1$ und $R^2$ jeweils eine niedere Alkyl-Gruppe oder eine niedere Alkoxy-Gruppe bezeichnen und

Y CH oder N bezeichnet,

dadurch gekennzeichnet, daß eine Verbindung der allgemeinen Formel (II)

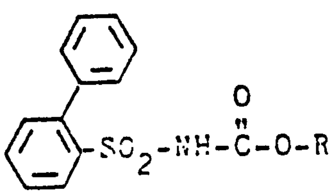

..... (II) ,

in der

R eine niedere Alkyl-Gruppe oder eine Aryl-Gruppe bezeichnet, mit einer Verbindung der allgemeinen Formel (III)

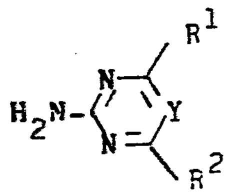

..... (III)

,

in der $R^1$, $R^2$ und Y die im Vorstehenden angegebenen Bedeutungen haben, umgesetzt wird.

2. Biphenylylsulfonylcarbamat-Derivate der allgemeinen Formel (II)

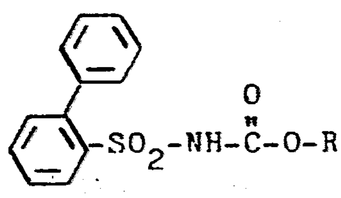

..... (II)

,

in der R eine niedere Alkyl-Gruppe oder eine Aryl-Gruppe bezeichnet.

3. Phenyl-2-biphenylylsulfonylcarbamat nach Anspruch 2 mit der nachstehenden Formel

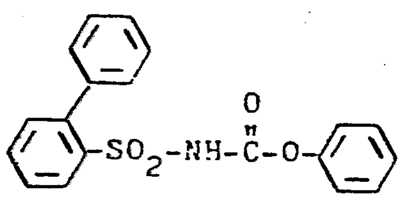

.

4. Verfahren zur Herstellung eines Biphenylylsulfonylcarbamat-Derivats der allgemeinen Formel (II)

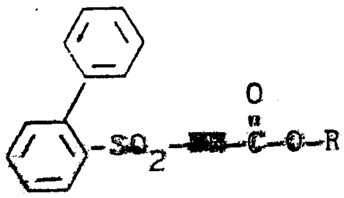

..... (II) ,

in der R eine niedere Alkyl-Gruppe oder eine Aryl-Gruppe bezeichnet, dadurch gekennzeichnet, daß 2-Phenylbenzolsulfonamid der folgenden Formel (V)

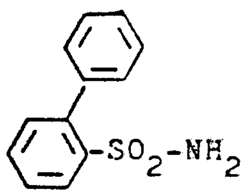

..... (V)

mit einer Verbindung der folgenden Formel (IV)

$$R'-\overset{\overset{O}{\|}}{C}-O-R \qquad \text{..... (IV)}$$

in der R die im Vorstehenden angegebene Bedeutung hat und R' ein Chlor-Atom oder die Gruppe -O-R bezeichnet, in der R die im Vorstehenden angegebene Bedeutung hat, umgesetzt wird.